# EUROPEAN PATENT APPLICATION

(11) **EP 4 220 651 A1**
(43) Date of publication of application: **02.08.2023**
(21) Application number: 23154570.8
(22) Date of filing: 01.02.2023
(51) Int. Cl.: G16H 10/20, G06N 20/00

(54) **ELECTRONIC PLATFORM FOR PRESENTING BURDEN SCORES FOR PARTICIPATION IN A CLINICAL TRIAL**

(30) Priority: 01.02.2022 IN 202211005448
(71) Applicant: ZS Associates, Inc., Evanston, IL 60201 (US)
(72) Inventor: BAGGA, Abhishek, Hitchin (GB); GROVE, Nicholl, Guildford (GB); OLAH, Zachary, Brooklyn (US); PRASHAD, Garima, New Delhi (IN)
(74) Representative: Murgitroyd & Company

(57) **Abstract**

Disclosed herein are methods and systems to predict, quantify, and present a patient's burden when participating in a clinical trial. A method includes gathering data associated with a clinical trial, such as receiving operational parameters of a new clinical trial. The method also includes executing one or more computer models to predict a quantified burden associated with the clinical trial including a patient burden score, a site burden score, and/or a burden cost. The method then includes presenting an interactive electronic platform that includes one or more graphical user interfaces configured to present the patient burden score(s) where when one or more operational attributes of the clinical trial is revised, the electronic platform revises the presented data accordingly.

## Description

### CROSS REFERENCE TO RELATED APPLICATIONS

This application claims the benefit of and priority to Indian Provisional Application No. 202,211,005,448, filed on February 1, 2022, which is incorporated herein in its entirety for all purposes.

### TECHNICAL FIELD

This application relates generally to generating, training, calibrating, and executing computer models to quantify and predict a patient burden score and to populate graphical user interfaces accordingly.

### BACKGROUND

Forecasting patient burden is conventionally a very slow, expensive, inefficient, and inaccurate process. Conventionally, a team of human analysts requests feedback from a representative sample of patients who have participated in clinical trials. The human analysis may then use their subjective skills and understanding (and sometimes conventional computer-implemented methods, such as spreadsheets) to determine a burden associated with the clinical trial. Not only is this process tedious, time-consuming, and expensive, it is also unreliable because the results depend directly on the human reviewer's subjective skills and understanding.

During the past several years, drug developers in the public and private sectors have expressed keen interest in the systematic measurement of participation burden in clinical trials. The demand to quantify participation burden is due to a number of factors including rising investment in patient-centric development planning and execution and the intensifying efforts to manage the ongoing adverse impact of protocol complexity on clinical trial timeliness, efficiency, and cost.

### SUMMARY

For the aforementioned reasons, there is a need to remove the subj ectivity of the conventional approaches and to intelligently and empirically measure the patient burden of a clinical trial. There is a need to develop computer models that can quantify and predict patient burden scores, in a manner that is more accurate than conventional methods.

There is also a need to present and visualize the calculated/predicted patient burden scores for a clinical trial in a manner that can be easily understood by end-users. While some conventional methods provide static platforms that display a patient's burden, the methods and systems discussed herein provide an interactive electronic platform where the patient burden is displayed using a variety of visual elements to provide a holistic snapshot for clinical trials. Specifically, the graphical user interfaces (GUIs) discussed herein can visualize the patient burdens (patient burden score, site burden score, and patient cost burden) when compared to other clinical trials.

The interactive electronic platform discussed herein can ingest clinical trial information from the end-user, display patient burden scores for different portions of the clinical trial using various visual aids displayed on different GUIs and easy-to-understand graphs, allow the end-user to revise one or more attributes of the clinical trial, and dynamically revise the GUIs accordingly. As a result, the end-user can estimate how and why patients are predicted to have high or low burdens for the clinical trial.

The interactive electronic platform provided using the methods and systems discussed herein is also referred to herein as the Study Design Optimizer (SDO) platform. Using the SDO platform, end-users are able to optimize operational parameters of a clinical trial or clinical study.

As used herein, a patient burden is a standard, quantified metric used across clinical development decision-making to inform patient-focused trial design, predict trial performance, and improve the trial patient experience. The methods and systems described herein predict the patient burden to understand the patient experience during an actual or simulated clinical trial and to establish a link between patient burden and clinical trial performance outcomes to accelerate the adoption of patient-focused clinical trials (e.g., drug development) across the industry.

Using the methods and systems discussed herein, a patient burden calculation and presentation system can utilize one or more computer models that implement a comprehensive participant burden algorithm based on protocol procedures, participation requirements, and lifestyle preferences of patients/participants to predict a burden score for patients and participants of a clinical trial. The system can survey data associated with various clinical trials and can analyze and generate the algorithm accordingly. Specifically, the system can train artificial intelligence (AI) and machine learning (ML) models to uncover hidden patterns and make connections between a participant's burden and various operational parameters of a clinical trial, which were previously impractical and not feasible using conventional methods.

Using the methods and systems disclosed herein, the system can also perform descriptive statistics, significance tests, and univariate analyses to ensure the accuracy and fitness of the model. Using the methods and systems described herein, strong statistically significant associations can be established between the participant burden algorithm and protocol performance outcomes including cycle times, number of amendments, enrollment rate, patient retention and screen failure rates.

The present disclosure presents an advancement in computer modeling and empirical analysis of quantifying participation burden that will assist clinicians (e.g., drug development teams) and protocol authors in retrospectively understanding clinical trial performance outcomes and in prospectively informing protocol design decisions

In an embodiment, a method comprises receiving, by a processor, a set of operational parameter attributes associated of a clinical trial; executing, by the processor, a computer model to calculate a patient burden score, a site burden score, and a cost burden quantifying a patient's burden and experience participating in the clinical trial; and populating, by the processor, a set of visual elements within a graphical user interface, such that the visual elements indicate the patient burden score, the site burden score, and the cost burden compared to a range of patient burden scores, site burden scores, or the cost burdens from a set of historical clinical studies.

The set of historical clinical studies may share a common operational parameter attribute with the clinical trial.

The operational parameter may be at least one of timing, medications, lab tests, blood tests, examinations, non-invasive procedures, invasive procedure, imaging procedure burden, or self-assessment questionnaire burden.

The patient burden score may correspond to at least one of demographics, participation logistics, lifestyle factors, caregiver involvement, and procedural burden of a patient associated with the clinical trial.

Receiving the at least one attribute of the clinical trial may correspond to extracting, by the processor, the at least one attribute from an electronic document.

The visual element corresponds to a procedure performed during the clinical study.

The visual element may include an indicator for a mean or medium value for patient burden scores, site burden scores, or the cost burdens of the set of historical clinical studies.

The set of operational parameter attributes associated of the clinical trial may correspond to a set of endpoints for the clinical trial.

In another embodiment, a system comprises a server comprising a processor and a non-transitory computer-readable medium containing instructions that when executed by the processor causes the processor to perform operations comprising receive a set of operational parameter attributes associated of a clinical trial; execute a computer model to calculate a patient burden score, a site burden score, and a cost burden quantifying a patient's burden and experience participating in the clinical trial; and populate a set of visual elements within a graphical user interface, such that the visual elements indicate the patient burden score, the site burden score, and the cost burden compared to a range of patient burden scores, site burden scores, or the cost burdens from a set of historical clinical studies.

The set of historical clinical studies may share a common operational parameter attribute with the clinical trial.

The operational parameter may be at least one of timing, medications, lab tests, blood tests, examinations, non-invasive procedures, invasive procedure, imaging procedure burden, or self-assessment questionnaire burden.

The patient burden score corresponds to at least one of demographics, participation logistics, lifestyle factors, caregiver involvement, and procedural burden of a patient associated with the clinical trial.

Receiving the at least one attribute of the clinical trial may correspond to extracting, by the processor, the at least one attribute from an electronic document.

The visual element may correspond to a procedure performed during the clinical study.

The visual element may include an indicator for a mean or medium value for patient burden scores, site burden scores, or the cost burdens of the set of historical clinical studies.

The set of operational parameter attributes associated of the clinical trial corresponds to a set of endpoints for the clinical trial.

In another embodiment, a system comprises a server in communication with an electronic device configured to display a graphical user interface, the server configured to receive a set of operational parameter attributes associated of a clinical trial; execute a computer model to calculate a patient burden score, a site burden score, and a cost burden quantifying a patient's burden and experience participating in the clinical trial; and populate a set of visual elements within the graphical user interface, such that the visual elements indicate the patient burden score, the site burden score, and the cost burden compared to a range of patient burden scores, site burden scores, or the cost burdens from a set of historical clinical studies.

The set of historical clinical studies may share a common operational parameter attribute with the clinical trial.

The operational parameter may be at least one of timing, medications, lab tests, blood tests, examinations, non-invasive procedures, invasive procedure, imaging procedure burden, or self-assessment questionnaire burden.

The patient burden score may correspond to at least one of demographics, participation logistics, lifestyle factors, caregiver involvement, and procedural burden of a patient associated with the clinical trial.

### BRIEF DESCRIPTION OF THE DRAWING FIGURES

Objects, aspects, features, and advantages of embodiments disclosed herein will become more fully apparent from the following detailed description, the appended claims, and the accompanying drawing figures in which reference numerals identify similar or identical elements. Reference numerals that are introduced in the specification in association with a drawing figure may be repeated in one or more subsequent figures without additional description in the specification to provide context for other features, and not every element may be labeled in every figure. The drawing figures are not necessarily to scale, emphasis instead being placed upon illustrating embodiments, principles, and concepts. The drawings are not intended to limit the scope of the claims included herewith.
**FIG. 1A** is a block diagram of embodiments of a computing device;
**FIG. 1B** is a block diagram depicting a computing environment comprising client devices in communication with cloud service providers;
**FIG. 2** is a block diagram of an example system in which performance prediction management services may manage and streamline access by clients to resource feeds (via one or more gateway services) and/or software-as-a-service (SaaS) applications;
**FIG. 3** is an example computing environment for the patient burden calculation and presentation system, in accordance with one or more implementations;
**FIGS. 4A-F** illustrate examples of workflow executed by the patient burden calculation and presentation system, in accordance with one or more implementations;
**FIGS. 5A-B** illustrate non-limiting examples of workflow executed by the patient burden calculation and presentation system, in accordance with one or more implementations; and
**FIGS. 6A-6O** illustrate non-limiting examples of GUIs of the SDO platform, in accordance with one or more implementations.

The features and advantages of the present solution will become more apparent from the detailed description set forth below when taken in conjunction with the drawings, in which like reference characters identify corresponding elements throughout. In the drawings, reference numbers generally indicate identical, functionally similar, and/or structurally similar elements.

### DETAILED DESCRIPTION

Reference will now be made to the illustrative embodiments depicted in the drawings, and specific language will be used here to describe the same. It will nevertheless be understood that no limitation of the scope of the claims or this disclosure is thereby intended. Alterations and further modifications of the inventive features illustrated herein, and additional applications of the principles of the subject matter illustrated herein, which would occur to one skilled in the relevant art and having possession of this disclosure, are to be considered within the scope of the subject matter disclosed herein. Other embodiments may be used and/or other changes may be made without departing from the spirit or scope of the present disclosure. The illustrative embodiments described in the detailed description are not meant to be limiting of the subject matter presented.
Section A describes a computing environment that may be useful for practicing embodiments described herein;
Section B describes a non-limiting example of a patient burden calculation and presentation system;
Section C describes non-limiting examples of methods to develop and implement an algorithm to predict patient burden scores; and
Section D describes non-limiting examples of one more GUIs presenting the patient burden scores for a clinical trial.

### Section A: Computing Environment:

Prior to discussing the specifics of embodiments of the systems and methods of an appliance and/or client, it may be helpful to discuss the computing environments in which such embodiments may be deployed.

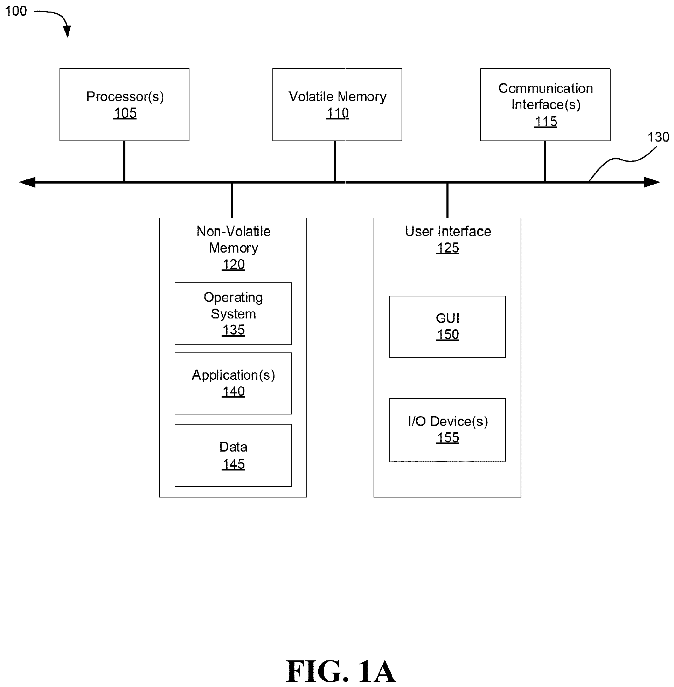

As shown in **FIG. 1A****,** computer **100** may include one or more processors **105,** volatile memory **110** (e.g., random access memory (RAM)), non-volatile memory **120** (e.g., one or more hard disk drives (HDDs) or other magnetic or optical storage media, one or more solid-state drives (SSDs) such as a flash drive or other solid-state storage media, one or more hybrid magnetic and solid-state drives, and/or one or more virtual storage volumes, such as cloud storage, or a combination of such physical storage volumes and virtual storage volumes or arrays thereof), user interface (UI) **125,** one or more communications interfaces **115,** and communication bus **130.** User interface **125** may include a graphical user interface (GUI) **150** (e.g., a touchscreen, a display, etc.) and one or more input/output (I/O) devices **155** (e.g., a mouse, a keyboard, a microphone, one or more speakers, one or more cameras, one or more biometric scanners, one or more environmental sensors, one or more accelerometers, etc.). The non-volatile memory **120** stores operating system **135,** one or more applications **140,** and data **145** such that, for example, computer instructions of operating system **135** and/or applications **140** are executed by processor(s) **105** out of volatile memory **110.** In some embodiments, volatile memory **110** may include one or more types of RAM and/or a cache memory that may offer a faster response time than the main memory. Data may be entered using an input device of GUI **150** or received from I/O device(s) **155.** Various elements of computer **100** may communicate via one or more communication buses, shown as communication bus **130.**

Computer **100** as shown in **FIG. 1A** is shown merely as an example, as clients, servers, intermediary, and other networking devices and may be implemented by any computing or processing environment and with any type of machine or set of machines that may have suitable hardware and/or software capable of operating as described herein. Processor(s) **105** may be implemented by one or more programmable processors to execute one or more executable instructions, such as a computer program, to perform the functions of the system. As used herein, the term "processor" describes circuitry that performs a function, an operation, or a sequence of operations. The function, operation, or sequence of operations may be hardcoded into the circuitry or soft coded by way of instructions held in a memory device and executed by the circuitry.

A "processor" may perform the function, operation, or sequence of operations using digital values and/or using analog signals. In some embodiments, the "processor" can be embodied in one or more application-specific integrated circuits (ASICs), microprocessors, digital signal processors (DSPs), graphics processing units (GPUs), microcontrollers, field-programmable gate arrays (FPGAs), programmable logic arrays (PLAs), multi-core processors, or general-purpose computers with associated memory. The "processor" may be analog, digital or mixed-signal. In some embodiments, the "processor" may be one or more physical processors or one or more "virtual" (e.g., remotely located or "cloud") processors. A processor including multiple processor cores and/or multiple processors may provide functionality for parallel, simultaneous execution of instructions, or for parallel, simultaneous execution of one instruction on more than one piece of data.

Communications interfaces **115** may include one or more interfaces to enable computer **100** to access a computer network such as a Local Area Network (LAN), a Wide Area Network (WAN), a Personal Area Network (PAN), or the Internet through a variety of wired and/or wireless or cellular connections.

In described embodiments, the computing device **100** may execute an application on behalf of a user of a client computing device. For example, the computing device 100 may execute a virtual machine, which provides an execution session within which applications execute on behalf of a user or a client computing device, such as a hosted desktop session. The computing device **100** may also execute a terminal services session to provide a hosted desktop environment. The computing device **100** may provide access to a computing environment including one or more of one or more applications, one or more desktop applications, and one or more desktop sessions in which one or more applications may execute.

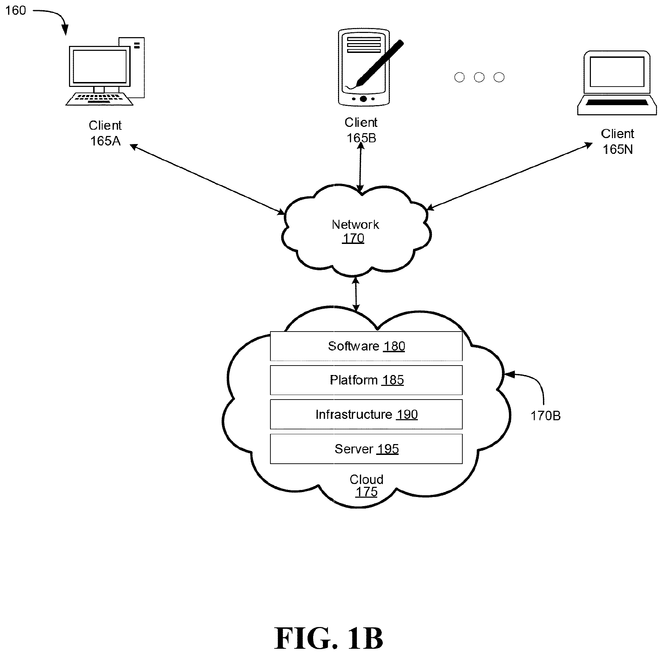

Referring to **FIG. 1B****,** a computing environment **160** is depicted. Computing environment **160** may generally be implemented as a cloud computing environment, an on-premises ("on-prem") computing environment, or a hybrid computing environment including one or more on-prem computing environments and one or more cloud computing environments. When implemented as a cloud computing environment, also referred to as a cloud environment, cloud computing, or cloud network, computing environment **160** can provide the delivery of shared services (e.g., computer services) and shared resources (e.g., computer resources) to multiple users. For example, the computing environment **160** can include an environment or system for providing or delivering access to a plurality of shared services and resources to a plurality of users through the internet. The shared resources and services can include but are not limited to, networks, network bandwidth, servers, processing, memory, storage, applications, virtual machines, databases, software, hardware, analytics, and intelligence.

In some embodiments, the computing environment **160** may provide client **165** with one or more resources provided by a network environment. The computing environment **160** may include one or more clients **165a-165n**, in communication with a cloud **175** over one or more networks **170.** Clients **165** may include, e.g., thick clients, thin clients, and zero clients. The cloud **108** may include back-end platforms, e.g., servers, storage, server farms, or data centers. The clients **165** can be the same as or substantially similar to computer **100** of **FIG. 1A****.**

The users or clients **165** can correspond to a single organization or multiple organizations. For example, the computing environment **160** can include a private cloud serving a single organization (e.g., enterprise cloud). The computing environment **160** can include a community cloud or public cloud serving multiple organizations. In some embodiments, the computing environment **160** can include a hybrid cloud that is a combination of a public cloud and a private cloud. For example, the cloud **175** may be public, private, or hybrid. Public clouds **108** may include public servers that are maintained by third parties to the clients **165** or the owners of the clients **165.** The servers may be located off-site in remote geographical locations as disclosed above or otherwise. Public clouds **175** may be connected to the servers over a public network **170.** Private clouds **175** may include private servers that are physically maintained by clients **165** or owners of clients **165.** Private clouds **175** may be connected to the servers over a private network **170.** Hybrid clouds **175** may include both the private and public networks **170** and servers.

The cloud **175** may include back-end platforms, e.g., servers, storage, server farms, or data centers. For example, the cloud **175** can include or correspond to a server or system remote from one or more clients **165** to provide third-party control over a pool of shared services and resources. The computing environment **160** can provide resource pooling to serve multiple users via clients **165** through a multi-tenant environment or multi-tenant model with different physical and virtual resources dynamically assigned and reassigned responsive to different demands within the respective environment. The multi-tenant environment can include a system or architecture that can provide a single instance of the software, an application, or a software application to serve multiple users. In some embodiments, the computing environment **160** can provide on-demand self-service to unilaterally provision computing capabilities (e.g., server time, network storage) across a network for multiple clients 165. The computing environment **160** can provide elasticity to dynamically scale out or scale in responsive to different demands from one or more clients **165.** In some embodiments, the computing environment **160** can include or provide monitoring services to monitor, control, and/or generate reports corresponding to the provided shared services and resources.

In some embodiments, the computing environment **160** can include and provide different types of cloud computing services. For example, the computing environment **160** can include Infrastructure as a service (IaaS). The computing environment **160** can include Platform as a service (PaaS). The computing environment **160** can include server-less computing. The computing environment **160** can include Software as a service (SaaS). For example, the cloud **175** may also include a cloud-based delivery, e.g., Software as a Service (SaaS) **180,** Platform as a Service (PaaS) **185,** and Infrastructure as a Service (IaaS) 190. IaaS may refer to a user renting the use of infrastructure resources that are needed during a specified time period. IaaS providers may offer storage, networking, servers, or virtualization resources from large pools, allowing the users to quickly scale up by accessing more resources as needed. Examples of IaaS include AMAZON WEB SERVICES provided by Amazon.com, Inc., of Seattle, Washington; RACKSPACE CLOUD provided by Rackspace US, Inc., of San Antonio, Texas; Google Compute Engine provided by Google Inc. of Mountain View, California; or RIGHTSCALE provided by RightScale, Inc., of Santa Barbara, California. PaaS providers may offer functionality provided by IaaS, including, e.g., storage, networking, servers, or virtualization, as well as additional resources such as, e.g., the operating system, middleware, or runtime resources. Examples of PaaS include WINDOWS AZURE provided by Microsoft Corporation of Redmond, Washington; Google App Engine provided by Google Inc.; and HEROKU provided by Heroku, Inc., of San Francisco, California. SaaS providers may offer the resources that PaaS provides, including storage, networking, servers, virtualization, operating system, middleware, or runtime resources. In some embodiments, SaaS providers may offer additional resources including, e.g., data and application resources. Examples of SaaS include GOOGLE APPS provided by Google Inc.; SALESFORCE provided by Salesforce.com Inc. of San Francisco, California; or OFFICE 365 provided by Microsoft Corporation. Examples of SaaS may also include data storage providers, e.g., DROPBOX provided by Dropbox, Inc., of San Francisco, California; Microsoft SKYDRIVE provided by Microsoft Corporation; Google Drive provided by Google Inc.; or Apple ICLOUD provided by Apple Inc. of Cupertino, California.

Clients **165** may access IaaS resources with one or more IaaS standards, including, e.g., Amazon Elastic Compute Cloud (EC2), Open Cloud Computing Interface (OCCI), Cloud Infrastructure Management Interface (CIMI), or OpenStack standards. Some IaaS standards may allow clients access to resources over HTTP and may use Representational State Transfer (REST) protocol or Simple Object Access Protocol (SOAP). Clients **165** may access PaaS resources with different PaaS interfaces. Some PaaS interfaces use HTTP packages, standard Java APIs, JavaMail API, Java Data Objects (JDO), Java Persistence API (JPA), Python APIs, web integration APIs for different programming languages including, e.g., Rack for Ruby, WSGI for Python, or PSGI for Perl, or other APIs that may be built on REST, HTTP, XML, or other protocols. Clients **165** may access SaaS resources through the use of web-based user interfaces, provided by a web browser (e.g., GOOGLE CHROME, Microsoft INTERNET EXPLORER, or Mozilla Firefox provided by Mozilla Foundation of Mountain View, California). Clients **165** may also access SaaS resources through smartphone or tablet applications, including, e.g., Salesforce Sales Cloud or Google Drive app. Clients **165** may also access SaaS resources through the client operating system, including, e.g., Windows file system for DROPBOX.

In some embodiments, access to IaaS, PaaS, or SaaS resources may be authenticated. For example, a server or authentication server may authenticate a user via security certificates, HTTPS, or API keys. API keys may include various encryption standards such as e.g., Advanced Encryption Standard (AES). Data resources may be sent over Transport Layer Security (TLS) or Secure Sockets Layer (SSL).

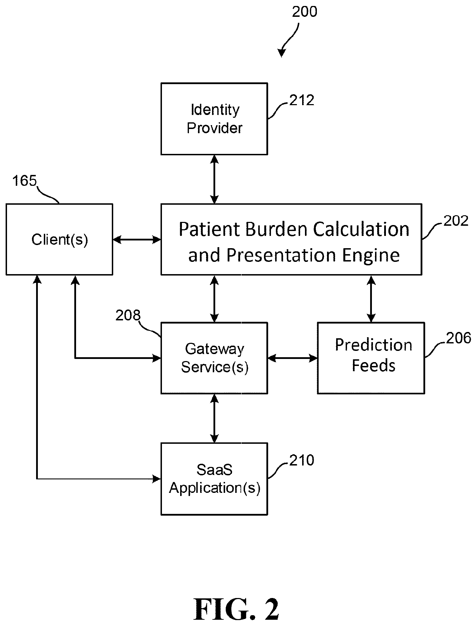

**FIG. 2** is a block diagram of an example system **200** in which an a patient burden calculation and presentation engine **202** may manage and streamline access by one or more clients **165** to one or more prediction feeds **206** (via one or more gateway services **208**) and/or one or more software-as-a-service (SaaS) applications **210.** As used herein, a prediction feed is a result of the execution of one or more AI models discussed herein. In particular, the patient burden calculation and presentation engine **202** may employ an identity provider **212** to authenticate the identity of a user of a client **165** and, following authentication, identify one or more prediction feeds the user is authorized to access. For the prediction feed(s) **206,** the client **165** may input attributes associated with a clinical trial and may request access to one or more AI models via a gateway service **208.** For the SaaS application(s) **210,** the client **165** may access the selected application directly. The SaaS application(s) **210** may allow the client **165** to access the SDO platform discussed herein and view the prediction feeds **206.**

The client(s) **165** may be any type of computing device capable of accessing the prediction feed(s) **206** and/or the SaaS application(s) **210,** and may, for example, include a variety of desktop or laptop computers, smartphones, tablets, etc. Each of the patient burden calculation and presentation engine **202,** the prediction feed(s) **206,** the gateway service(s) **208,** the SaaS application(s) **210,** and the identity provider **212** may be located within an on-premises data center of an organization for which the system **200** is deployed, within one or more cloud computing environments, or elsewhere.

### Section B: Patient Burden Calculation and Presentation System

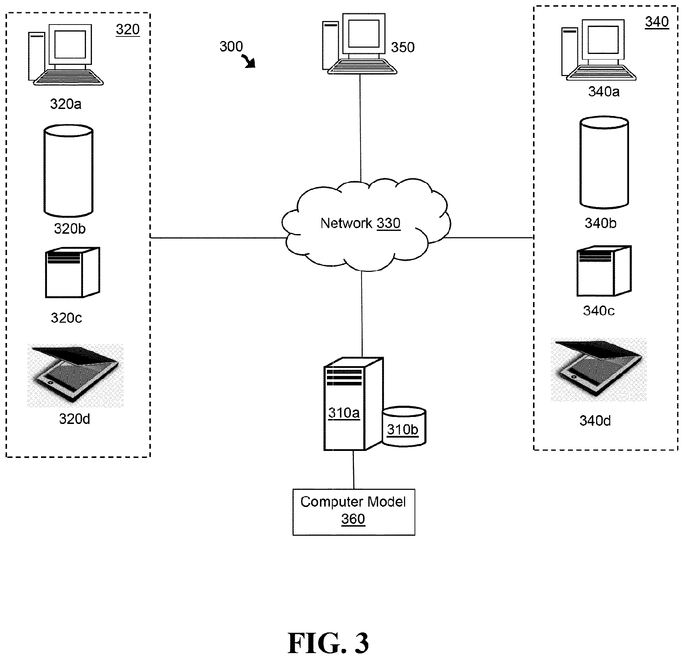

As will be described throughout, a server of patient burden calculation and presentation system **300** (such as an analytics server **310a**) can retrieve and analyze data using various methods described herein to calculate a patient burden score and present the calculations in a manner that is easily consumable and customizable for end-users. **FIG. 3** is a non-limiting example of components of the patient burden calculation and presentation system **300** in which the analytics server **310a** operates. The analytics server may be any computer, server, or processor described in **FIGS. 1A-2****.**

The analytics server **310a** may utilize features described in **FIG. 3** to retrieve data and to generate/display results. The analytics server **310a** is communicatively coupled to a system database **310b,** electronic data sources **320a-d** (collectively electronic data sources **320**), end-user devices **340a-d** (collectively end-user device **340**), and an administrator computing device **350.** The system **300** is not confined to the components described herein and may include additional or alternative components, not shown for brevity, which is to be considered within the scope of the embodiments described herein.

The above-mentioned components may be connected through a network **330.** The examples of the network **330** may include but are not limited to, private or public LAN, WLAN, MAN, WAN, and the Internet. The network **330** may include both wired and wireless communications according to one or more standards and/or via one or more transport mediums.

The analytics server **310a** may utilize one or more application programming interfaces (APIs) to communicate with one or more of the electronic devices described herein. For instance, the analytics server may utilize APIs to automatically receive data from the electronic data sources **320.** The analytics server **310a** can receive data as it is generated, monitored, and/or processed by the electronic data source **320.** For instance, the analytics server **110a** may utilize an API to receive clinical trial data from the database **320b** without any human intervention. This automatic communication allows for faster retrieval and processing of data.

The analytics server **310a** may generate and/or host an electronic platform (also referred to as the SDO platform) having a series of graphical user interfaces (GUIs) configured to use various computer models to project and display data associated with a clinical trial. The SDO platform can be displayed on the electronic data sources **320,** the administrator computing device **350,** and/or end-user devices **340.** An example of the SDO platform generated and/or hosted by the analytics server **310a** may be a web-based application or a website configured to be displayed on different electronic devices, such as mobile devices, tablets, personal computers, and the like. Even though certain embodiments discuss the analytics server **310a** displaying the results, it is expressly understood that the analytics server **310a** may either directly generate and display the SDO platform described herein or may present the data to be presented on a GUI displayed on the end-user devices **340.**

The analytics server **310a** may host a website (also referred to herein as the SDO platform or electronic platform) accessible to end-users operating any of the electronic devices described herein (e.g., end-users), where the content presented via the various webpages may be controlled based upon each particular user's role or viewing permissions. The analytics server **310a** may be any computing device comprising a processor and non-transitory machine-readable storage capable of executing the various tasks and processes described herein. Non-limiting examples of such computing devices may include servers, computers, workstation computers, personal computers, and the like. While this example of the system **300** includes a single analytics server **310a,** in some configurations, the analytics server **310a** may include any number of computing devices operating in a distributed computing environment.

The analytics server **310a** may execute one or more software applications configured to display the SDO platform (e.g., host a website), which may generate and serve various webpages to each electronic data sources **320** and/or end-user devices **340.** Different end-users may use the website to view and/or interact with the predicted results.

The analytics server **310a** may be configured to require user authentication based upon a set of user authorization credentials (e.g., username, password, biometrics, cryptographic certificate, and the like). In such implementations, the analytics server **310a** may access the system database **310b** configured to store user credentials, which the analytics server **310a** may be configured to reference to determine whether a set of entered credentials (purportedly authenticating the user) match an appropriate set of credentials that identify and authenticate the user.

The analytics server **310a** may also store data associated with each user operating one or more electronic data sources **320** and/or end-user devices **340.** The analytics server **310a** may use the data to determine whether a user device is authorized to view results generated computer model(s) discussed herein, such as the computer model **360.**

The computer model **360** may be any collection of one or more algorithms and machine-readable code that can ingest data associated with a patient and/or a clinical trial and to predict a patient burden score. Accordingly, the computer model **360** may include a mathematical algorithm. Additionally or alternatively, the computer model **360** may represent an AI/ML model (e.g., neural network) that can be trained in accordance with data received from the electronic data sources **320** and/or end-user devices **340.** Specifically, the analytics server **310** may use the data collected from the electronic data sources **320** to generate a training dataset and further train the AI model **360** using various machine learning techniques (e.g., supervised, unsupervised, or semi-supervised).

The analytics server **310a** may receive data associated with a clinical trial from end-user devices **340** and/or electronic data sources **320.** The electronic data sources **320** may represent different databases or third-party vendors who possess medical data, marketing data, clinical trial data, and the like. For instance, the electronic data sources **320** may represent computers, databases, and servers of a medical provider that can provide additional information regarding a clinical trial.

The analytics server **310a** may use the data collected from the electronic data sources **320** and received from the end-user device **340** to execute the computer model **360.** The analytics server **310a** then displays the results via the SDO platform (e.g., GUIs) on the administrator computing device **350** or the end-user devices **340.**

The end-user devices **340** may be any computing device comprising a processor and a non-transitory machine-readable storage medium capable of performing the various tasks and processes described herein. Non-limiting examples of an end-user device may include workstation computers, laptop computers, tablet computers, and server computers. In operation, various end-users may use end-user devices **340** to access the SDO platform operationally managed by the analytics server **310a** to enter clinical trial information and view predicted/projected results.

The administrator computing device **350** may represent a computing device operated by a system administrator. The administrator computing device **350** may be configured to display retrieved data, in the form of results generated by the analytics server **110a,** where the system administrator can monitor various models utilized by the analytics server **110a,** review feedback, and modify various thresholds/rules described herein.

The analytics server **310a** may access, generate, and execute various computer models. Although the example system **300** depicts the computer model **360** stored on the analytics server **310a,** the AI models may be stored on another device or server (e.g., store locally or in cloud storage).

In operation, the analytics server **310a** may collect data associated with various clinical trials (e.g., operational parameters associated with the clinical trials) and other patient experiences from a variety of sources (e.g., from the electronic data sources **320** or from the patients directly via a global survey). The analytics server **310a** may then train the computer model **360** to develop an algorithm to calculate/predict a patient burden score. When the computer model **360** is trained, the analytics server **310** may implement the computer model, and allow end-users to use the computer model **360** to view results. For instance, an end-user may use any of the end-user devices **340** to access the SDO platform and enter attributes of a clinical trial. The analytics server **310a** may then execute the computer model **360** to calculate a patient burden score and may populate the SDO platform accordingly.

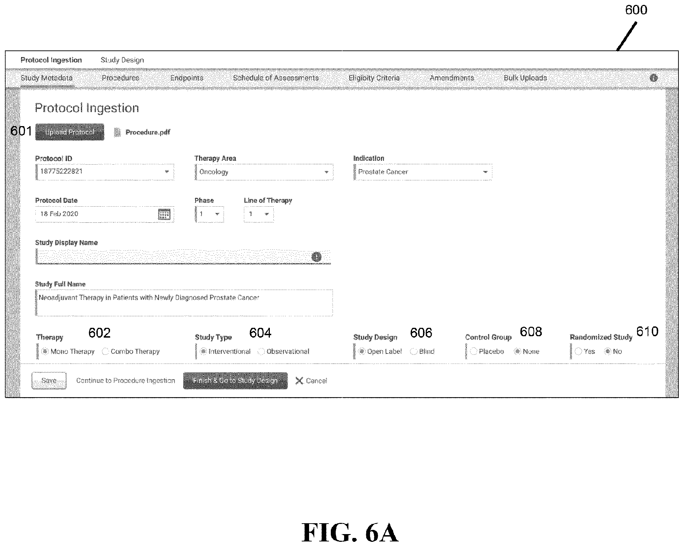

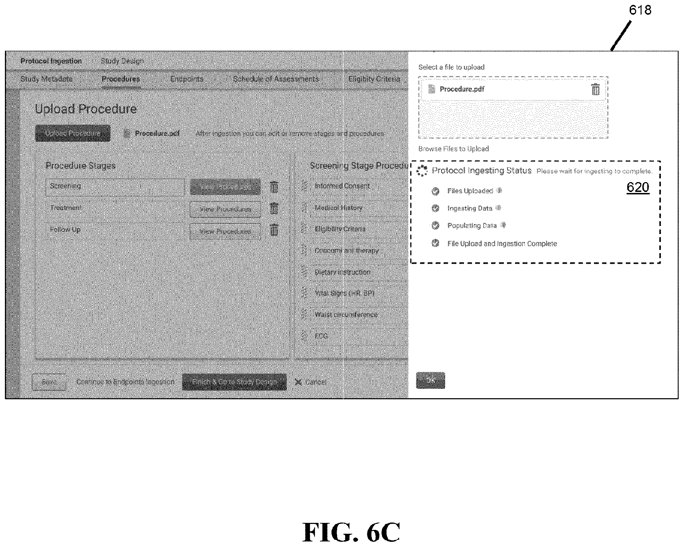

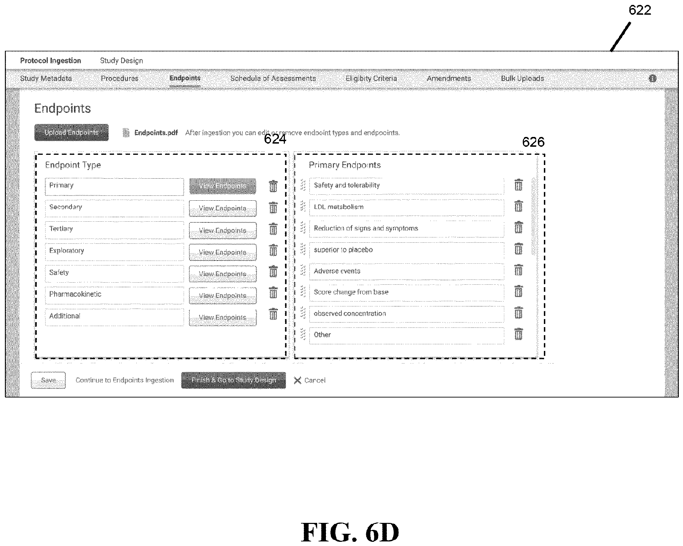

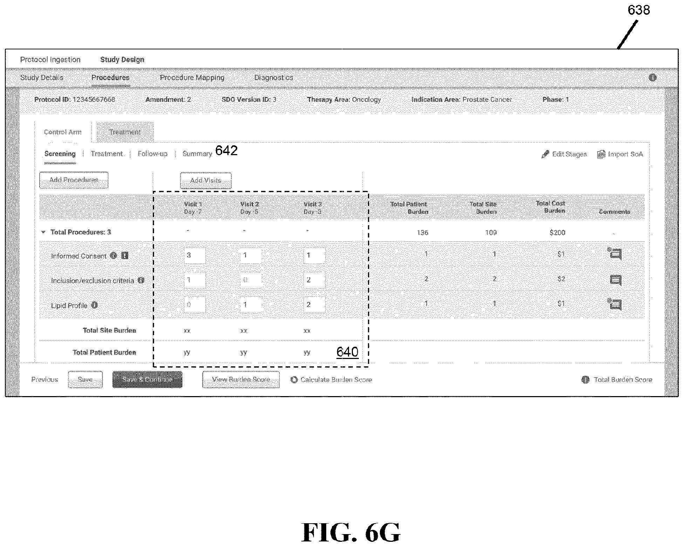

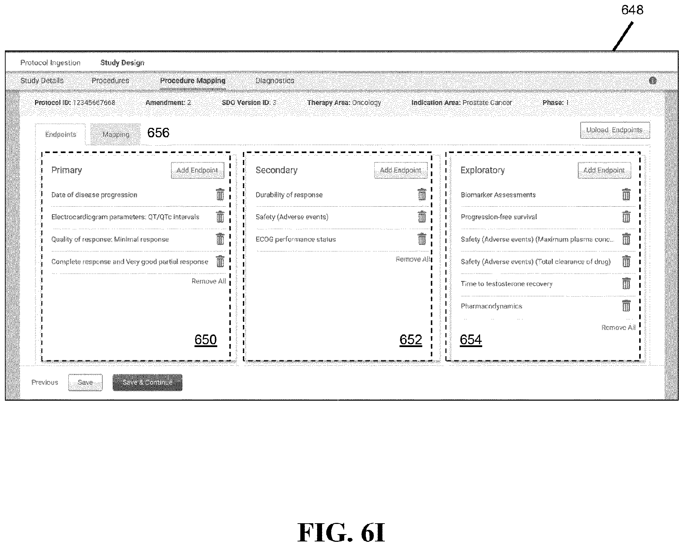

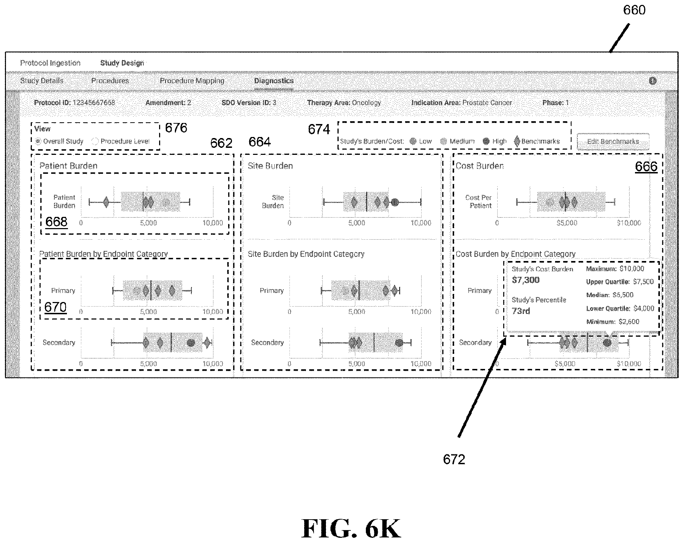

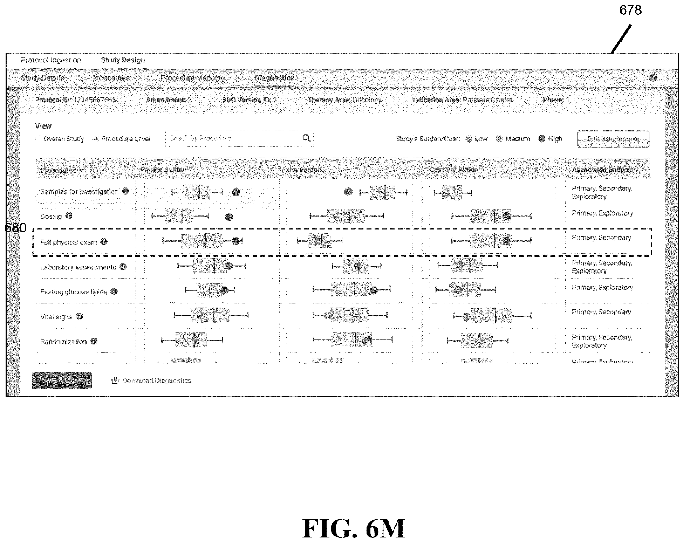

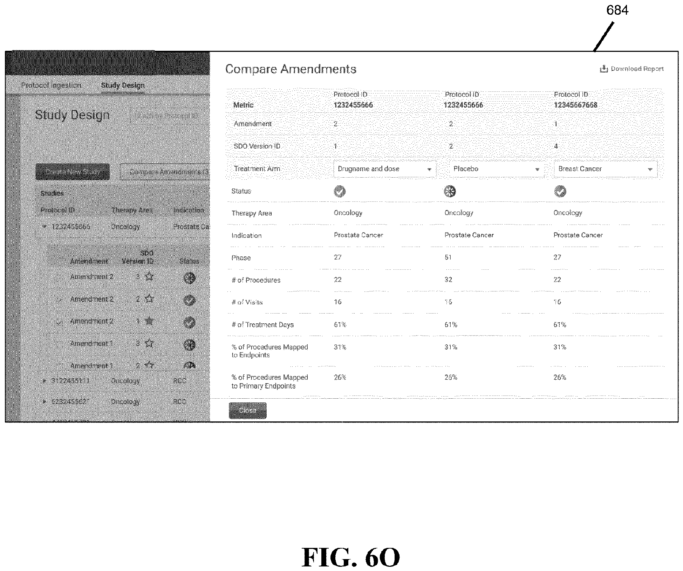

The SDO platform may include various GUIs discussed herein where each GUI may include various input elements allowing the end-user to input attributes of a clinical trial and see the results accordingly. An example of the GUIs presented within the SDO platform may include **FIGS. 6A-6N****.**

### Section C: non-limiting examples of methods to develop and implement an algorithm to predict a patient burden score:

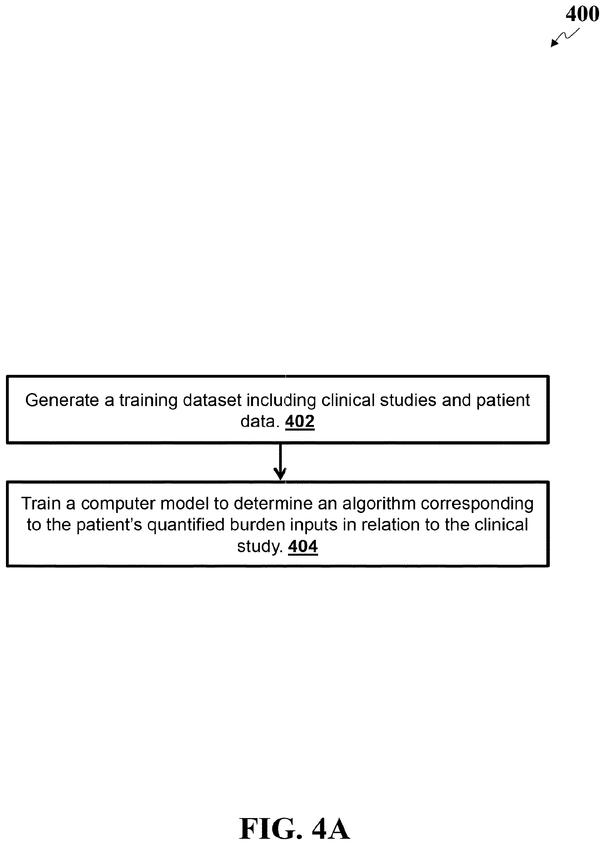

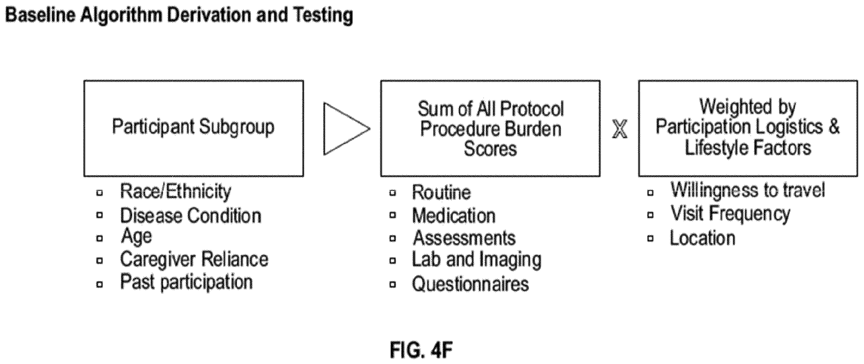

Referring now to **FIG. 4A****,** a workflow diagram for patient burden calculation and presentation system is depicted, in accordance with one or more implementations. The method **400** includes steps **402-404.** However, other embodiments may include additional or alternative execution steps or may omit one or more steps altogether. In addition, the method **400** is described as being executed by a system, similar to the system described in **FIG. 3****.** Different steps of the method **400** or different parts of the different steps may be executed by any number of computing devices operating in the distributed computing system described in **FIGS. 1A-3****.**

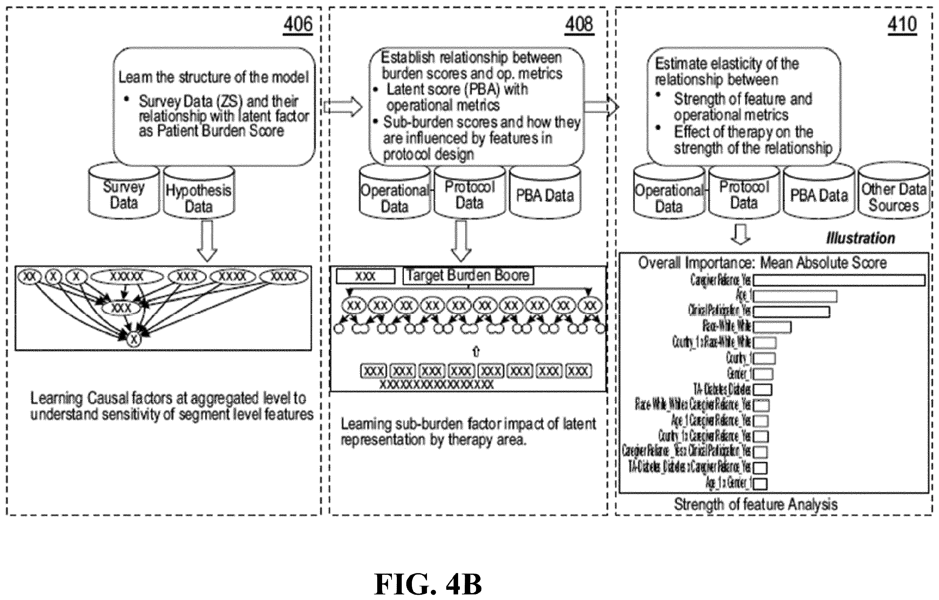

At step **402,** the analytics server may generate a training dataset including clinical study and patient data. Using the method **400,** the analytics server may generate and train a computer model that uses an enhanced baselined algorithm to quantify a patient burden and translate the burden to trial performance (e.g., evaluate new clinical studies). As depicted in **FIG. 4B****,** the analytics server may use a three-step process to understand how protocol design and patient attributes influence operational metrics and patient burden.

Specifically, the analytics server may generate and train a model (e.g., computer model **360**) to first learn the structure of the data and develop an algorithm to predict a patient burden score (step **406**). The analytics server may then establish a relationship between burden score and operational metrics (step **408**). For instance, the computer model may learn the sub-burden factor impact of latent representation by therapy area. Finally, at step **410**, the analytics server may examine the features within the data to identify/estimate an elasticity of the relationship between the strength of features and operational metrics and effects of therapy on the strength of the relationship.

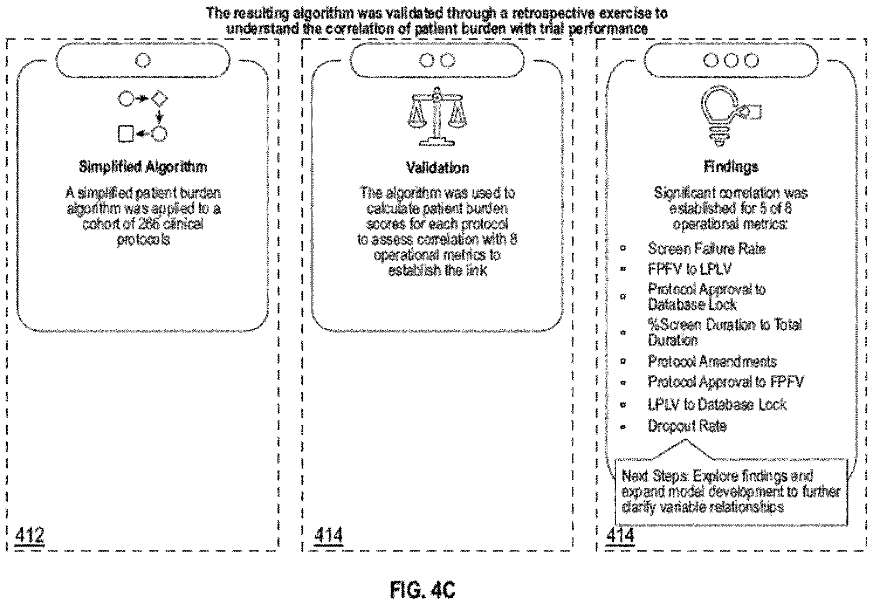

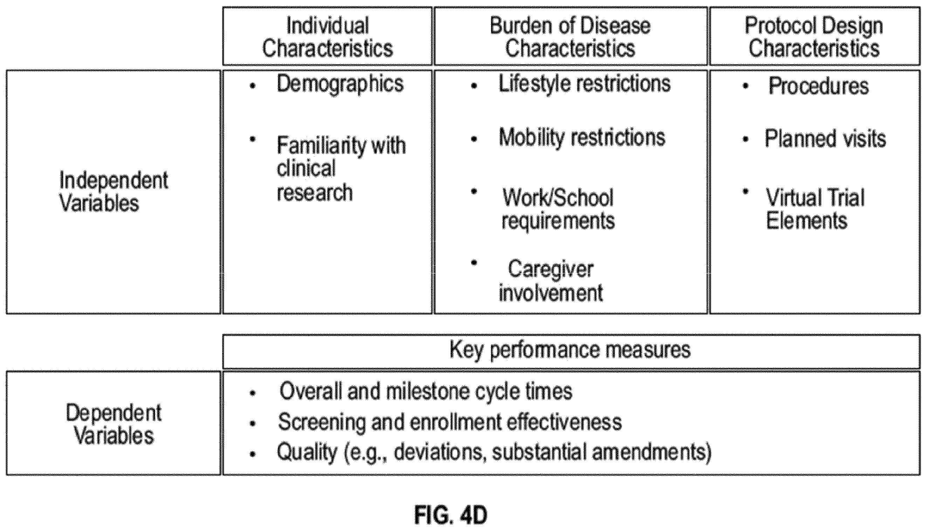

To develop the algorithm itself (e.g., to train the computer model), the analytics server may use a two-step process depicted in **FIG. 4C****.** The analytics server may first analyze data to generate the algorithm (**412**). When developing the algorithm, the analytics server may use a variety of factors (independent and dependent variables), as depicted in **FIG. 4D****.** Referring back to **FIG. 4C**, the analytics server may then test and validate the algorithm (**414**). Specifically, the analytics server may calculate a patient burden score for each protocol to assess correlation with different operational metrics and to determine if any connections among the data points exist. As a result, the analytics server may establish various connections and uncover patterns of how operational parameters of a clinical trial relate to patient burden and experiences as indicated by patient's responses and inputs.

To train the computer model and develop an algorithm that can predict the patient burden, the analytics server may first generate data associated with participants of previous clinical trials (e.g., training dataset). The analytics server may use the training dataset to develop the algorithm.

In order to retrieve pertinent data and to generate the training dataset, the analytics server may implement a global study and collect data associated with past patients and participants of a clinical trial. For instance, the analytics server may transmit interactive questionnaires to various computing devices associated with past participants of different clinical trials (e.g., electronic data sources **320** discussed in **FIG. 3**). In some configurations, an administrator (operating the administrator computer **350**) may conduct a large global online survey of individuals with and/or without prior clinical research participation experience. The analytics server may then aggregate, de-duplicate, analyze, and compile the perceived burden of procedures and participation conditions into scores, sensitive to variation by participant subgroup(s), thereby generating the training dataset. The analytics server may generate an algorithm accordingly and store the algorithm within a computer model (e.g., computer model **360**). The analytics server may also test and validate the algorithm and its ability to predict protocol performance outcomes.

The survey transmitted to the participants may incorporate questions that probe lifestyle factors, medication adherence burden, transportation and distance requirements, and remote and/or virtual study visit approaches. The analytics server may divide the survey instrument into five core sections: demographics, participation logistics, lifestyle factors, caregiver involvement, and procedural burden. The analytics server may analyze each category individually in order to identify an algorithm that can assess a patient's needs and predict a score indicating their burden in participating in a clinical trial.

The survey may include various input elements designed to receive input from the participants. For instance, perceived procedural burden may be assessed using the numerical scale (e.g., from 0-100) and a corresponding input element allowing the participants to input their responses. For this scale, respondents may be asked to compare the perceived procedural burden relative to the administration of procedures to gather vital signs (e.g., temperature, blood pressure, and pulse) in order to standardize scores across cultures and individual tolerances.

Once the data is received, the analytics server may aggregate the data into one or more training datasets. Referring back to **FIG**. **4A**, at step **404**, the analytics server may then train a computer model to generate an algorithm corresponding to the patient's quantified burden inputs. The algorithm, as used herein, may learn from the training dataset and may uncover patterns and links among data points within the training dataset to identify how a patient's experiences (e.g., indicated by the responses received from the patients) relate to the clinical study/trial.

The algorithm may derive protocol-specific dimensions associated with patient burden to analyze the data, such as procedural (e.g., effort, time commitment, anxiety and pain associated with each procedure), convenience (e.g., number of visits, travel distance, type of transportation, days of work missed, or arranging for child care), lifestyle (e.g., restrictions associated with diet, exercise, smoking, or alcohol consumption), caregiver involvement (e.g., enrolling in the study, recording data and notes, helping administer study drug, or providing transportation and child care), and the like.

When the algorithm is finalized, the computer model may then ingest new data (e.g., data associated with a new clinical study and/or a new patient participating in the new study) and may predict a burden sore for the new clinical study. For instance, the computer model can predict specific performance metrics (corresponding predicted patient burden) regarding screen failure rate, first patient first visit (FPFV) to last patient last visit (LPLV), protocol approval, percentage of screen to total duration, protocol amendments, protocol approval to FPFV, LPLV to database lock, and/or dropout rate.

The computer model may include any algorithm (whether utilizing AI/ML techniques or not). For instance, the computer model may be a mathematical algorithm that is not developed using AI/ML techniques. Additionally or alternatively, the computer model may comprise a neural network that is iteratively trained by the analytics server using the training dataset. In an alternative embodiment, the analytics server may use a gradient boosting method to train the computer model. However, it is expressly understood that the computer model is not limited to either method.

In various non-limiting embodiments, the computer model may use one or more deep learning engines to train itself. Although exemplified using deep neural networks, it should be understood that any alternative and/or additional deep learning model(s) might be used to implement deep learning engines. The deep learning engines include processing pathways that are trained during the training phase. A multi-layer neural network may consist of a stack of layers each performing a specific operation, e.g., pooling, loss calculation, etc. Each intermediate layer receives the output of the previous layer as its input. The beginning layer may be an input layer, which is directly connected to an input received from the training dataset. The next set of layers may be convolutional layers that present the results of convolving a certain number of filters with the input data and perform as a feature extractor. The output of each layer may be considered as an activation map, which highlights the effect of applying a specific analytical protocol on the input.

The analytics server may train the computer model using a supervised method where the data records within the training dataset are labeled. For instance, the training dataset may be labeled, such that the computer model can identify and distinguish which input corresponds to which operational parameter of the clinical study that was reviewed positively (or poorly) by one or more patients.

Additionally or alternatively, the analytics server may use an unsupervised method where the training dataset is not labeled. Because labeling the data within the training dataset may be time-consuming and may require vast computing power, the analytics server may utilize unsupervised training techniques to train the computer model. The analytics server may not be limited to the above-described AI/MI, training techniques. For instance, the analytics server may use both techniques, wherein the analytics server may label data when applicable and use a supervised training method (e.g., certain portions of the data are labeled as ground truth). If the analytics server cannot verify the accuracy of portions of the data retrieved, the analytics server may use an unsupervised training method. Therefore, the analytics server may use a semi-supervised method to train the computer model. Additionally or alternatively, the analytics server may also utilize reinforcement learning to train the computer model.

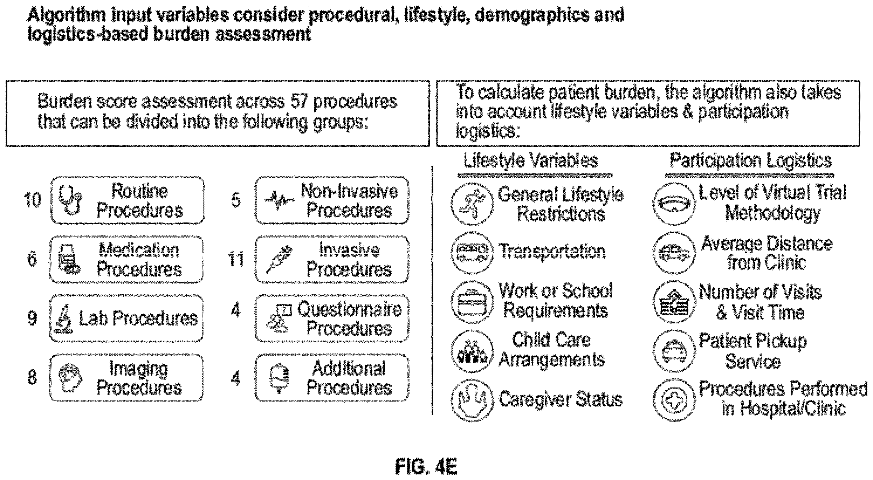

During training, the analytics server may manipulate the data and analyze different portions of the data. For instance, the computer model may use a data split to segment the data into different subgroups. As depicted in **FIG. 4E**, for different common procedures, the analytics server may divide the responses into eight procedure groups:
First: medications (6 procedures);
Second: lab and blood tests (9 procedures);
Third: routine examinations (10 procedures);
Fourth: non-invasive procedures (5 procedures);
Fifth: invasive procedures (11 procedures);
Sixth: imaging procedure burden (8 procedures);
Seventh: self-assessment questionnaire burden (4 procedures); and
Eighth: other common procedures (5 procedures).

The analytics server may also test and validate the algorithm to ensure its accuracy. Specifically, the analytics server may utilize previous studies assessing protocol design practices (e.g., various studies based on convenience samples of actual protocols provided by pharmaceutical, biopharmaceutical, and contract research organizations) to evaluate the model/algorithm's fitness. In a non-limiting example, de-identified protocols compiled by other organizations can be used to test and validate the participation burden algorithm.

The analytics server may iteratively refine the algorithm until and unless the algorithm satisfies one or more accuracy thresholds. When the algorithm satisfies the accuracy threshold, the computer model can be implemented to analyze new clinical trials and predict a corresponding patient burden score.

Because the computer model is iteratively trained, the algorithm can be refined with time, such that the algorithm is iteratively improved. Therefore, the algorithm may not be a static algorithm. That is, certain variables factors used in the algorithm may be refined to fit the data and to produce a more accurate patient burden score.

### Section D describes non-limiting examples of the SDO platform presenting the patient burden, cost burden, and/or site score for a clinical trial.

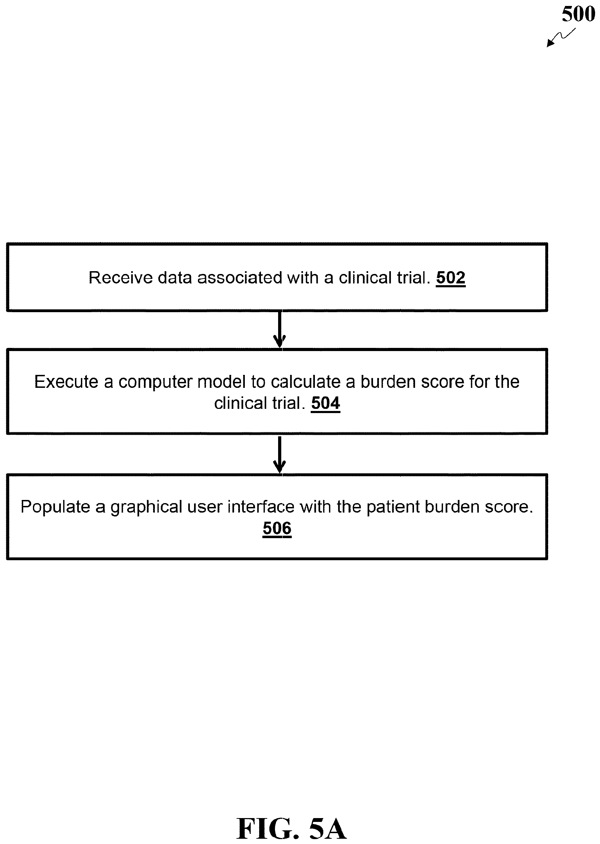

Referring now to **FIG. 5A**, a workflow diagram for patient burden calculation and presentation system is depicted, in accordance with one or more implementations. The method **500** includes steps **502-506.** However, other embodiments may include additional or alternative execution steps or may omit one or more steps altogether. In addition, the method **500** is described as being executed by a system, similar to the system described in **FIG. 3**. Different steps of the method **500** or different parts of the different steps may be executed by any number of computing devices operating in the distributed computing system described in **FIGS. 1A-3****.**

Using the method **500,** an end-user can develop a study proposal for a clinical trial in light of different therapeutic areas, intervention, procedures, endpoints, lifestyle variables, and participation logistics. Using the method **500,** a server, such as the analytics server depicted in **FIG. 3**, can provide an interactive platform (also referred to herein as the SDO platform) that allows end-users to benchmark their clinical protocols against historical clinical trial data to understand patient burden, site burden, and cost footprint of their new clinical trial design. Using the SDO platform end-users may reduce protocol complexity by eliminating non-essential procedures and identifying procedures that are most impactful (e.g., optimize the clinical trial procedures and timelines). Using the methods and systems described herein, the SDO platform can identify and classify the procedures in 'CARE' model which is Core, Auxiliary, Routine and Essential. This analysis leads to identification of non-essential procedures linked to exploratory end points.

The SDO platform introduces patient-centric approaches to protocol design of a clinical trial, which embraces voice of patient that has been codified and quantified using the computer modeling techniques described herein.

Using the method **500,** the SDO platform may collect study (i.e., clinical trial or clinical study) identification data (e.g., schedule of activities, general study details, therapy area, indications, patient segment, and/or phase, and the like). For instance, an end-user may enter all the study design elements (e.g., therapy, study type, study design, control group, and treatment arm structures) or may allow the SDO platform to automatically ingest documents and extract the data needed. The SDO platform may then create a schedule of assessments that includes data associated with screenings, treatment follow-up arms, add-in procedures, visit schedules, and procedure frequency. The SDO platform may allow end-users to view a summary of the schedule of assessments, view calculated patient, site, and/or cost burden scores.

At step **502**, the system may receive or retrieve data associated with a clinical trial. The system may provide the SDO platform that can collect details associated with a clinical trial and its procedures and/or map the pertinent data. As depicted and described in **FIGS**. **6A-6O**, the SDO platform may include various input elements that allow end-users to manually input attributes of the clinical trial. Additionally or alternatively, end-users can upload documents where the SDO platform atomically extracts and determines the pertinent information (e.g., attributes associated with the clinical trial).

The SDO platform can use various data analytics protocols (e.g., natural language processing (NLP), image recognition, and/or machine learning methods) to extract the relevant trial design information from protocol documents associated with a clinical trial (uploaded by an end-user).

For instance, the SDO platform may use NLP techniques and a data engineering pipeline to extract relevant trial design information from protocol documents provided by the end-user. Once the data is retrieved, the SDO platform may analyze the extracted design attributes with respect to the patient burden, site burden, and client's internal cost benchmarks using the computer model discussed herein. The SDO platform allows for the comparison of multiple design options to create "what-if" scenarios and to optimize protocol design.

As used herein, data associated with the clinical trial may refer to any data associated with the clinical trial. For instance, the user may (either directly or via uploading proper documents) input operational parameters associated with the clinical trial.

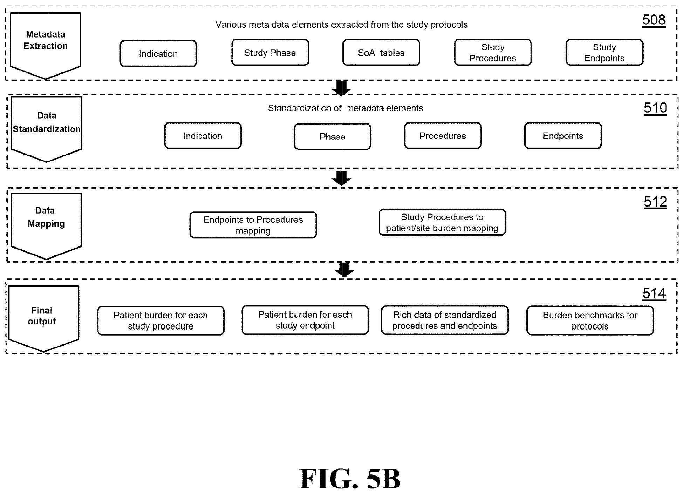

Referring now to **FIG. 5B**, a schematic design of data ingestion and analysis by the system is illustrated. As depicted in **508**, when the system receives a document and analyzes the document (using image recognition and NLP protocols), the system may also extract pertinent metadata from the documents that may indicate various attributes of the clinical trial (e.g., indications, study phases, schedule of assessment tables, study procedures, and endpoints). As depicted in **510**, the system may then standardize the data using a standard table. For instance, the system may use standard terminology (used by any particular medical organization) the standardize the extracted elements. As depicted in **512**, the system may then use various data mapping protocols to map the extracted and identified data to different endpoints or procedures. Using the data mapping, the system can determine which data (extracted from the uploaded documents) corresponds to which part of the clinical trial (e.g., which procedure, endpoint, or phase of the clinical trial).

Referring back to **FIG. 5A**, at step **504**, the system may execute a computer model to calculate a burden score for the clinical trial. The system may execute the computer model discussed herein (e.g., computer model **360** in **FIG. 3** and developed as discussed in section C) to identify a patient burden, cost burden, and/or a site burden for the clinical trial. The computer model may be a previously trained AI/MI, model that can ingest attributes of the clinical trial (e.g., collect in the step **502**) and can predict the burden scores for the clinical study. The computer model discussed herein may utilize ML techniques and utilize training datasets that include codified and quantified patient burden data from a large number of global patients within various (e.g., five) therapeutic areas to develop a robust quantitative approach to protocol burden analysis.

The system may use the data retrieved to execute the computer model (e.g., trained using the methods and systems discussed herein) to identify a patient burden score, a site burden score, and a cot burden value associated with the clinical trial, such as different procedures, endpoints, and the like. For instance, the computer model may ingest the operational parameters (e.g., procedures, endpoints, requirements, and the like) associated with the clinical trial and compare those data points with the training data. Therefore, based on those data points, the system may predict a patient burden score, a cost, and site burden.

At step **506**, the system may populate one or more GUIs using the predicted burden scores (step **504**). The system may output the predicted results using one or more visual presentation methods. For instance, as depicted in **514**, the system may output burden scores for each study procedure or for each study endpoint. The system may also show burden scores for the clinical trial in comparison with other clinical trials (e.g., benchmark trials or historical trials).

In a non-limiting example, the system may display the SDO platform that includes GUIs depicted in **FIGS. 6A-6O****.** The SDO platform depicted in these figures is a non-limiting example of the electronic platform displayed by the system. Therefore, it is expressly understood that the SDO platform may include fewer or more GUIs and some displayed GUIs may display fewer or more features than shown in these figures. Therefore, several variations of the depicted GUIs are within the scope of the disclosed SDO platform.

The SDO platform may allow the end-user to manually input data associated with a new clinical trial or may allow the user to upload pertinent documents associated with a clinical trial (automatic ingestion), or both.

The SDO platform may start by displaying the GUI **600** (depicted in **FIG. 6A**). The GUI **600** is a protocol ingestion page that may include various input elements to collect data associated with a clinical trial, such as the protocol identifier, therapy area (e.g., oncology), indication (e.g., prostate cancer), protocol date (e.g., start date), protocol phase, and a line of therapy. The input elements may also include a full name and a display name for the clinical trial (or clinical study).

The GUI **600** may also include input elements (e.g., radio buttons) allowing the end-user to select whether the clinical trial is a monotherapy or a combo therapy (input element **602**), study type (international vs observational) as depicted in the input element **604,** open-label vs blind (input element **606**), control group as indicated by the input element **608** (placebo or not), and/or randomized study (input element **610**).

Instead of manually inputting the data using the input elements of the GUI **600,** the SDO platform may allow for automatic ingestion of data. For instance, the GUI **600** may also include the input element **601** that allows the end-user to upload a document associated with the clinical trial. When the SDO platform determines that the end-user has interacted with the input element **601,** the SDO platform may allow the end-user to upload a document.

Once the SDO platform determines that the end-user has uploaded a document, the system may use various protocols (e.g., NLP, AI/MI, modeling, and/or image recognition) to identify the pertinent data associated with the clinical trial. Additionally or alternatively, the system may analyze metadata associated with the uploaded document and extract/analyze said metadata. The system may use various computer models to identify the content of the uploaded documents and to map the extracted information to the correct category of the clinical trial. If the end-user has not manually entered the data depicted in **FIG. 6A****,** the SDO platform may automatically populate the relevant input elements depicted using the data from the uploaded documents.

After extracting the data, the system may also normalize the data. For instance, various terms may be extracted and standardized in accordance with common procedural terminology indicated by any medical association or organization or specific terminology used by the end-user or their institution (e.g., different institutions may have their own terminology or may use specific proprietary tables).

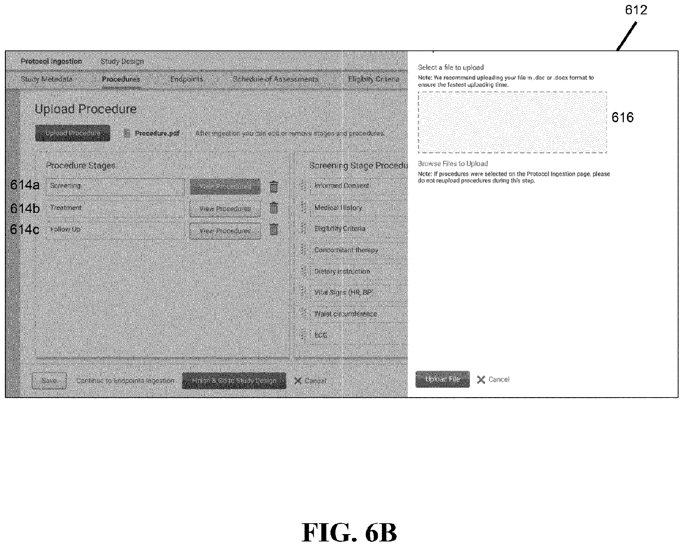

The SDO platform may also collect data associated with procedures of the new clinical trial, as depicted in **FIGS. 6B-C.** The GUI **612** may include data input elements **614a-**c to allow the end-user to upload documents associated with the clinical trial, such as procedural documents or any document that includes how the clinical trial is to be conducted (e.g., data associated with screening, treatment, and follow up).

The GUI **612** may also include input element **616** that allows the user to upload a document (e.g., either browse through files stored locally or in a shared data repository). Once the SDO platform determines that the end-user has uploaded a document, the system may use various protocols (e.g., NLP and/or image recognition) to identify the pertinent data associated with the clinical trial. When the system determines that the end-user has interacted with the input element **616,** the SDO platform may direct the user to the GUI **618** that provides an indication of the document uploaded to the end-user. The GUI **618** displays that the file has been uploaded and data has been ingested and populated (graphical element **620**).

The system may use various analytical methods to map out the procedures within the uploaded document. For instance, the SDO platform may automatically identify the protocol, a stage of the protocol (screening, treatment, and follow-up) for the extracted data. Therefore, using the GUIs depicted in **FIGS. 6B-C** the SDO platform can extract both the procedures of a clinical trial and their corresponding stages.

The SDO platform may also include a GUI **622** designated to receive endpoint information. Endpoint, as used herein, may refer to an event or outcome that can be measured objectively to determine whether the intervention being studied is beneficial. The endpoints of a clinical trial are usually included in the study objectives. Some examples of endpoints are survival, improvements in quality of life, relief of symptoms, or disappearance of the tumor.

Using the input elements depicted herein, the end-user may either input the endpoints or may instruct the SDO platform to extract the endpoints automatically. The endpoints may belong to different types (e.g., primary, secondary, tertiary, exploratory, safety, and/or pharmaco-kinetic), as indicated by input elements within the graphical element **624**. Additionally, the SDO platform may allow the user to define their own type of endpoint (e.g., designated as additional). The SDO platform may extract the data necessary to identify and map out each endpoint (if applicable) and populate the input elements accordingly, such that the end-user can approve or deny the extracted and identified values.

Each endpoint may include various specific markers. For instance, as depicted within the graphical element **626,** the SDO platform provides the following endpoints for the primary endpoint: safety and tolerability, LDL metabolism, reduction of signs and symptoms, superior to placebo, adverse events, score change from a baseline, observed concentration, and other. It is noted that the purpose of the Endpoints screen may be to outline the endpoints of the work-in-progress protocol. Therefore, the above-references examples refers to different non-limiting examples of different of types of primary endpoints. The end points can be used to assess whether the therapy is beneficial.

The end-user can review and determine whether the SDO's extraction and automatic identification is accurate. For instance, the end-user can determine whether the SDO platform has correctly identified the primary endpoints displayed within the graphical element **626.** In some configurations, certain endpoints may be described in the uploaded documents using free text, thereby analyzing the endpoints and automatically identifying the endpoint type may result in identifications that must be approved by end-users. The end-user can then revise, add, and/or remove any data extracted/identified by the SDO platform.

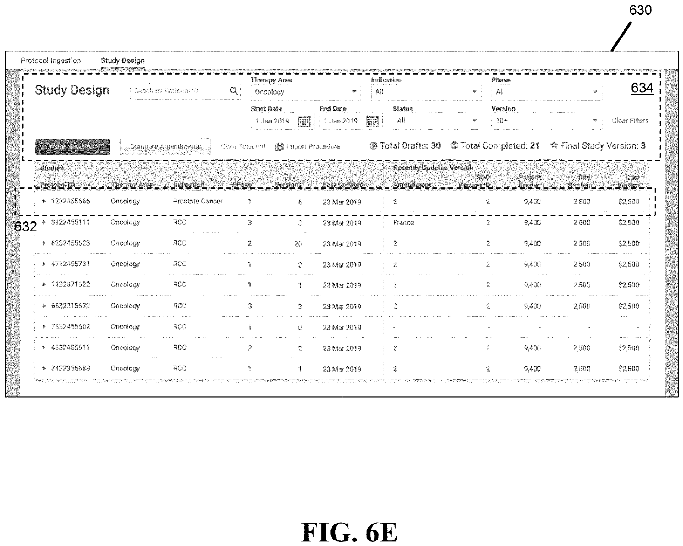

After the clinical data associated with the clinical trial has been inputted and/or uploaded by the end-user, the SDO platform directs the end-user towards GUI **630** (**FIG. 6E** or the study design page). The GUI **630** may be a dashboard that displays multiple clinical trials and brief information associated with each clinical trial. The user may use various input elements depicted within the graphical element **634** to identify, filter, and/or sort through a collection of clinical trials in accordance with various attributes.

Each row may correspond to a particular clinical study. The non-limiting example of a clinical trial inputted by the user using the features described in **FIGS. 6A-6D** is displayed in row **632.** For each clinical trial, the SDO platform may display protocol ID, therapy area, indication, phase, version, and date that information associated with the clinical trial was last updated, and the like. The SDO platform may also display a quantified patient burden, site burden, and cost burden associated with the clinical trial. The user may interact with each clinical trial depicted within the GUI **630** receive more detailed data associated with the selected clinical trial.

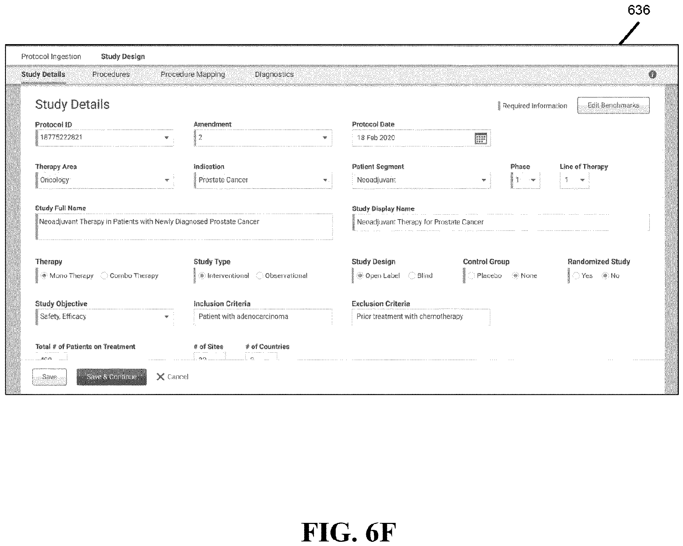

For instance, when the end-user selects the clinical trial depicted within the row **632,** the SDO platform directs the user to the GUI **636** (**FIG. 6F**). As depicted, the GUI **636** is similar to the GUI **600** because both GUIs depict various attributes of the clinical study. The end-user may use the information depicted within the GUI **636** to review and determine whether any attributes of the clinical trial were erroneously extracted and identified by the SDO platform. If any data point is incorrectly inputted and/or automatically ingested, the end-user may use various input elements depicted within the GUI **636** to revise the incorrect data points.

If the data associated with the clinical trial is correct and approved by the end-user, the SDO platform may direct the end-user to the GUI **638.** In the GUI **638,** the SDO platform displays a schedule of assessment for the clinical trial. As used herein, a schedule of assessment may refer to a representation of sequentially planned protocol events and activities of a particular clinical trial. For instance, the schedule of assessment may outline a plan of care that a typical participant will receive during his or her participation in the clinical trial.

The GUI **638** can be shown for either the control arm or the treatment arm. For brevity, the described embodiment only describes how data associated with the control arm is depicted. In any type of clinical trial (e.g., double-blinded placebo study), one might have multiple treatment arms or control arms and within each particular arm there may be a corresponding schedule that patients/participants, principle investigators, and clinical researchers consider when conducting the clinical trial and evaluating the patients (e.g., eligibility criteria and/or short/long term follow up). The GUI **638** displays a schedule associated with the procedures.

The GUI **638** may include various interactive buttons allowing the end-user to select a particular phase/stage of the clinical trial. For instance, the end-user may first control whether the displayed information belongs to the control arm or the treatment arm. Then, the end-user may select the procedure within the clinical trial (e.g., screening, treatment, follow-up, or summary). In this way, the end-user can see a subgroup or a particular portion of the procedures needed to be performed during the clinical trial. For instance, the depicted information belongs to the screening procedure of the control arm of the clinical trial.

For each portion of the procedures, the SDO platform may display a patient burden, site burden, and cost burden. For instance, as depicted, the schedule of assessment for the screening stage within the control arm is displayed in the GUI **638.** In this embodiment, the SDO platform has only identified three procedures for screening. However, other embodiments may include more or fewer procedures. The depicted procedures indicate that, when a patient starts the clinical trial, the patient must undergo an informed consent process and then inclusion/exclusion process based on various criteria (e.g., patient attributes that could exclude them from participating in the clinical trial). As depicted, patients must then go through a lipid profile test. The SDO platform displays how (e.g., timeline) these procedures must be performed as well. For instance, the graphical element **640** depicts that there are three different visits within that screening period. This indicates that these procedures are being conducted on three separate days.

These visits may affect the patient burden scores (site, cost, and/or patient burden). For instance, each row (which represents a subgroup of procedures) may display its own group of burdens (e.g., patient burden, site burden, and cost burden). The SDO platform may also display a total burden score for each category of burden scores (e.g., total site burden, total patient burden, and/or total cost burden).

Using the input elements depicted within the graphical element **640,** the end-user may revise the timeline associated with the procedures and determine if/how the revisions affect the burden scores. For instance, using the input elements within the graphical element **640,** the end-user may revise the timeline and the system may execute the computer model and re-calculate the patient burden using the revised data. As a result, the SDO platform may dynamically change the patient burden and the end-user can optimize the timeline for the procedure, such that the timeline reduces the amount of burden (or produces a minimum amount of burden) on patients. For instance, the end-user may combine the procedures, such that all three procedures are performed in one visit. As a result, the SDO platform may indicate that the patient burden decreases.

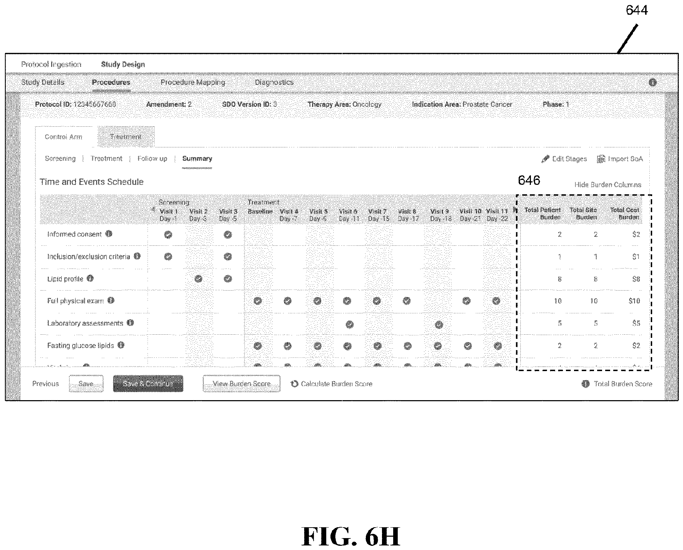

When the end-user interacts with the input element **642**, the SDO platform directs the end-user to the GUI **644**, depicted in **FIG. 6H**. The GUI **644** includes a summary of all three procedures (screening, treatment, and follow-up) and shows a visual timeline of when each procedure (or a specific part of the procedure) must be performed. The GUI **644** also includes a total burden (patient, site, and cost) for each procedure and/or each part of each procedure, as depicted in the graphical element **646**.

The SDO platform may also include an endpoint mapping GUI, as depicted in FIG. 61. The SDO may display a GUI dedicated to endpoints to show how the procedures and endpoints are related.

The **FIG. 61** displays the associated primary, secondary, and exploratory endpoints which will be used for procedure to endpoint mapping. Specifically, the GUI **648** shows different graphical elements (**650, 652,** and **654**) that correspond to different endpoints either directly inputted by the end-user or automatically identified by the SDO platform. The SDO platform displays the endpoints to allow the end-user to review and ensure that the correct procedures correspond to appropriate endpoints. The end-user can use a variety of input elements to add, remove, and/or revise any procedure from the endpoints.

Procedures, as used herein, may refer to various procedures performed on the patient where the data is collected and eventually analyzed for the purposes of evaluating the patient and the clinical trial itself. Therefore, the data collected is used to support the endpoints within the clinical trial. The GUI **648** displays procedures to endpoint mapping. Specifically, it displays which procedures support which endpoints. Thereby, the end-user can easily view and revise as needed, such that proper procedures are supporting the right endpoints.

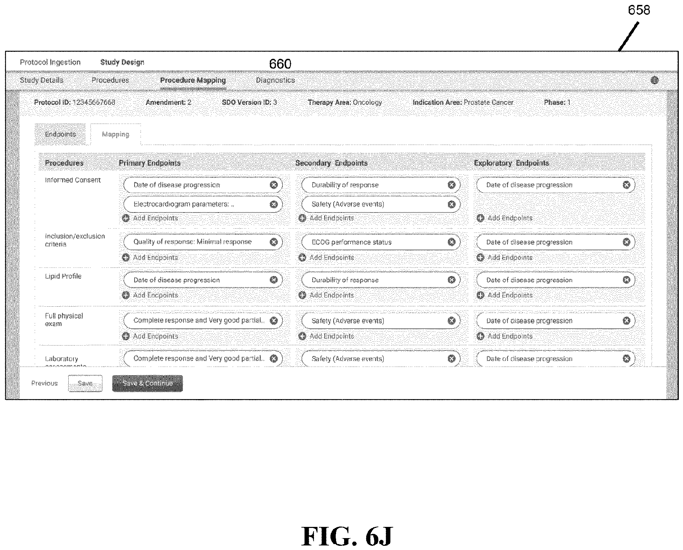

The SDO platform may also include an endpoint mapping GUI, as depicted in **FIG. 6J**. The SDO may display a GUI dedicated to endpoints to show how the procedures and endpoints are related. The SDO platform may also display procedures and schedules assessments and how they relate to different endpoints (e.g., mapping GUI). For instance, when the SDO platform determines that the end-user has interacted with the interactive element **656**, the SDO platform directs the end-user towards the GUI **658**. The GUI **658** allows the end-user to add, remove, or revise procedures to be performed for each endpoint. In the depicted embodiment, certain endpoints are dropped which are related to endpoint outside of primary/ secondary/ exploratory e.g. safety etc.

Once the end-user has finished reviewing and/or revising various data points defining the clinical trial, the end-user can view various diagnostics where the SDO platform analyzes the clinical trial using the methods and systems described herein (e.g., by executing the computer model). When the SDO platform determines that the user has interacted with the diagnostic interactive button **660,** the SDO platform a direct user to the GUI **662** where various burdens are displayed.

The GUI **660** includes three different graphical elements **662**, **664**, and **666**. Each graphical element displays a visual representation of a burden calculated using the methods and systems described herein. For instance, the graphical element **662** visualizes the patient burden, the graphical element **664** visualizes the site burden, and the graphical element **666** visualizes the cost burden of the clinical trial. Each graphical element may include one or more visual representations of a corresponding burden compared to one or more historical clinical trials used as benchmarks. Benchmark, as used herein, may refer to a clinical trial that is similar (share at least one attribute) to the clinical trial being analyzed and visualized by the SDO platform. For instance, the benchmark may be a clinical trial analyzing the same type of procedure or drug. For instance, benchmarks may serve to compare and contrast previously conducted trials within similar TA, indication, phase, line of therapy, route of administration, procedures, sponsor, etc. In some embodiments, the benchmark (e.g., historical study) may be similar to the clinical trial being analyzed because both are regarding the same disease. For instance, two trials/studies may be compared if they are both regarding prostate cancer.

In another example, the benchmark may correspond to a key clinical trial or a clinical trial performed by a competitor or a different department.

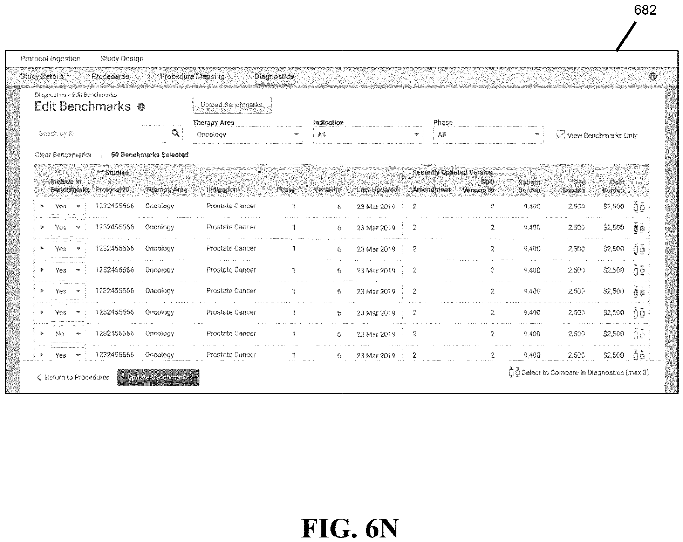

In some configurations, the benchmark may be automatically selected by the system or may be received from an end-user. The number of benchmarks may be revised by the user. For instance, as depicted in the GUI **660**, the end-user has selected three benchmarks. This allows the end-user to view the clinical trial in comparison to multiple benchmarks, which may be beneficial to the end-user. However, the end-user can increase or decrease the number of benchmarks. Optionally, the SDO platform may allow the end-user to select the benchmarks. For instance, the SDO platform may direct the end-user to GUI **682** (**FIG. 6N**) where the end-user can select one or more historical clinical trials as benchmarks.

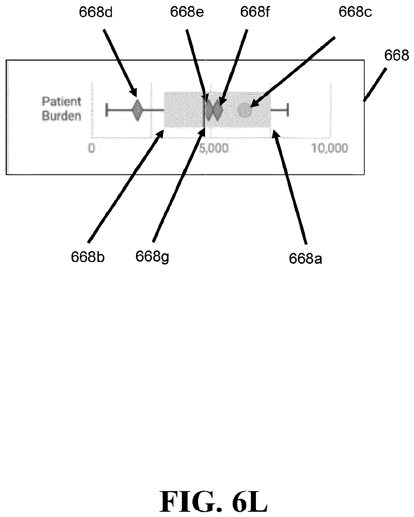

The visual indicators of the GUI **660** may provide a visual comparison of the clinical trial being analyzed by the end-user with similar historical clinical trials. Referring now to **FIG. 6L**, the visual indicator **668** is depicted. The visual indicator **668** visually represents the patient burden for the clinical study/trial. The SDO platform may indicate a range of the patient burden using the range of **668a-b** (e.g., indicated as a colored area in the depicted embodiment; however, in other embodiments, the range can be visually highlighted using any method). In some configurations, the range may correspond to all the similar clinical trials (e.g., similar to the clinical trial being analyzed) and their corresponding burden.

The SDO platform may also indicate the patient burden for similar benchmark clinical trials using the indicators **668d-f.** The indicator **668c,** however, represents the patient burden for the instant clinical trial (i.e., the clinical trial being analyzed by the end-user). The indicator **668c** may be visually distinct depending on its classification. For instance, the SDO platform may classify the patient burden (using various pre-defined thresholds) as low, medium, or high. The SDO platform may then display the patient burden indicator, such that it visually corresponds to its classification (e.g., using coloring schemes or shapes to indicate the burden's classification). In the depicted embodiment, the patient burden is shown as a colored circle, which corresponds to the map **674** in the GUI **660.** The visual indicator **668g** may also represent a mean or median of the patient burden within the dataset, such that the user can compare the burden associated with the instant study (indicator **668c**) with the mean (indicator **668g**). Using the depicted range, benchmark indicators, and the mean value, the end-user can visually identify that the clinical trial has a burden score that is within a realm of comparable clinical trials (within the third quartile) and not an outlier.

The SDO platform may display various visual indicators that are specific to a particular endpoint (e.g., visual indicator **670**) or the clinical trial as a whole (e.g., visual indicator **668**). The SDO platform may display visual indicators for site burden and cost burden using similar methods as the patient burden. The visual indicators may be interactive and may provide additional information upon the end-user's request. For instance, when the end-user interacts with a visual indicator (e.g., hovers over a visual indicator), the SDO platform may display the pop-up window **672** that displays detailed information and describes why the SDO platform has predicted a particular burden.

As indicated by the toggle **676,** the GUI **660** is visualizing overall burdens (patient, site, and cost) for the clinical trial. When the SDO platform determines that the end-user has interacted with the toggle **676** (indicating a desire to see burden visualization at a more granular procedural level), the SDO platform may direct the end-user to the GUI **678.**

The GUI **678** may include visual indicators that are similar to the visual indicators of the GUI **660.** However, the visual indicators within the GUI **678** correspond to different procedure levels (e.g., different procedures within the schedule of assessments). Therefore, each row indicates a particular procedure, and three visual indicators visualizing patient, site, and cost burden levels associated with that procedure. Each row may also include an associated endpoint. For instance, row **680** includes a visual indicator for the patient burden, site burden, and cost burden of full physical exam, which belongs to the primary and secondary endpoints. In this way, different procedures of the clinical trial can be evaluated individually.

As discussed herein, the SDO platform allows the end-user to revise various attributes of the clinical trial to analyze and optimize the clinical trials. For instance, the end-user may revise a timeline or a procedure and the SDO platform may dynamically revise the burden scores and visualizations accordingly. Moreover, the end-user may generate multiple versions of the same clinical trial and compare them separately. The GUI **684** may provide a holistic comparison between different versions (or amended) clinical trials. Using this data, the end-user may determine how to amend/optimize different attributes of the clinical trial.

The SDO platform discussed herein may include one or more of the GUIs discussed and depicted in **FIGS. 6A-6O**. However, it is expressly understood that the SDO platform is not limited to the features depicted and discussed herein.

The foregoing method descriptions and the process flow diagrams are provided merely as illustrative examples and are not intended to require or imply that the steps of the various embodiments must be performed in the order presented. The steps in the foregoing embodiments may be performed in any order. Words such as "then," "next," etc. are not intended to limit the order of the steps; these words are simply used to guide the reader through the description of the methods. Although process flow diagrams may describe the operations as a sequential process, many of the operations can be performed in parallel or concurrently. In addition, the order of the operations may be re-arranged. A process may correspond to a method, a function, a procedure, a subroutine, a subprogram, and the like. When a process corresponds to a function, the process termination may correspond to a return of the function to a calling function or a main function.

The various illustrative logical blocks, modules, circuits, and algorithm steps described in connection with the embodiments disclosed herein may be implemented as electronic hardware, computer software, or combinations of both. To clearly illustrate this interchangeability of hardware and software, various illustrative components, blocks, modules, circuits, and steps have been described above generally in terms of their functionality. Whether such functionality is implemented as hardware or software depends upon the particular application and design constraints imposed on the overall system. Skilled artisans may implement the described functionality in varying ways for each particular application, but such implementation decisions should not be interpreted as causing a departure from the scope of this disclosure or the claims.

Embodiments implemented in computer software may be implemented in software, firmware, middleware, microcode, hardware description languages, or any combination thereof. A code segment or machine-executable instructions may represent a procedure, a function, a subprogram, a program, a routine, a subroutine, a module, a software package, a class, or any combination of instructions, data structures, or program statements. A code segment may be coupled to another code segment or a hardware circuit by passing and/or receiving information, data, arguments, parameters, or memory contents. Information, arguments, parameters, data, etc. may be passed, forwarded, or transmitted via any suitable means including memory sharing, message passing, token passing, network transmission, etc.

The actual software code or specialized control hardware used to implement these systems and methods is not limiting of the claimed features or this disclosure. Thus, the operation and behavior of the systems and methods were described without reference to the specific software code being understood that software and control hardware can be designed to implement the systems and methods based on the description herein.

When implemented in software, the functions may be stored as one or more instructions or code on a non-transitory computer-readable or processor-readable storage medium. The steps of a method or algorithm disclosed herein may be embodied in a processor-executable software module, which may reside on a computer-readable or processor-readable storage medium. A non-transitory computer-readable or processor-readable media includes both computer storage media and tangible storage media that facilitate transfer of a computer program from one place to another. A non-transitory processor-readable storage media may be any available media that may be accessed by a computer. By way of example, and not limitation, such non-transitory processor-readable media may comprise RAM, ROM, EEPROM, CD-ROM or other optical disk storage, magnetic disk storage or other magnetic storage devices, or any other tangible storage medium that may be used to store desired program code in the form of instructions or data structures and that may be accessed by a computer or processor. Disk and disc, as used herein, include compact disc (CD), laser disc, optical disc, digital versatile disc (DVD), floppy disk, and Blu-ray disc where disks usually reproduce data magnetically, while discs reproduce data optically with lasers. Combinations of the above should also be included within the scope of computer-readable media. Additionally, the operations of a method or algorithm may reside as one or any combination or set of codes and/or instructions on a non-transitory processor-readable medium and/or computer-readable medium, which may be incorporated into a computer program product.

The preceding description of the disclosed embodiments is provided to enable any person skilled in the art to make or use the embodiments described herein and variations thereof. Various modifications to these embodiments will be readily apparent to those skilled in the art, and the principles defined herein may be applied to other embodiments without departing from the spirit or scope of the subject matter disclosed herein. Thus, the present disclosure is not intended to be limited to the embodiments shown herein but is to be accorded the widest scope consistent with the following claims and the principles and novel features disclosed herein.

While various aspects and embodiments have been disclosed, other aspects and embodiments are contemplated. The various aspects and embodiments disclosed are for purposes of illustration and are not intended to be limiting, with the true scope and spirit being indicated by the following claims.

## Claims

1. A method comprising:
receiving, by a processor, a set of operational parameter attributes associated of a clinical trial;
executing, by the processor, a computer model to calculate a patient burden score, a site burden score, and a cost burden quantifying a patient's burden and experience participating in the clinical trial; and
populating, by the processor, a set of visual elements within a graphical user interface, such that the visual elements indicate the patient burden score, the site burden score, and the cost burden compared to a range of patient burden scores, site burden scores, or the cost burdens from a set of historical clinical studies.

2. The method of claim 1, wherein the set of historical clinical studies share a common operational parameter attribute with the clinical trial.

3. The method of claim 1, wherein the operational parameter is at least one of timing, medications, lab tests, blood tests, examinations, non-invasive procedures, invasive procedure, imaging procedure burden, or self-assessment questionnaire burden.

4. The method of claim 1, wherein the patient burden score corresponds to at least one of demographics, participation logistics, lifestyle factors, caregiver involvement, and procedural burden of a patient associated with the clinical trial.

5. The method of claim 1, wherein receiving the at least one attribute of the clinical trial corresponds to extracting, by the processor, the at least one attribute from an electronic document.

6. The method of claim 1, wherein the visual element corresponds to a procedure performed during the clinical study.

7. The method of claim 1, wherein the visual element includes an indicator for a mean or medium value for patient burden scores, site burden scores, or the cost burdens of the set of historical clinical studies.

8. The method of claim 1, wherein the set of operational parameter attributes associated of the clinical trial corresponds to a set of endpoints for the clinical trial.

9. A system comprising:
a server comprising a processor and a non-transitory computer-readable medium containing instructions that when executed by the processor causes the processor to perform operations comprising:
receive a set of operational parameter attributes associated of a clinical trial;
execute a computer model to calculate a patient burden score, a site burden score, and a cost burden quantifying a patient's burden and experience participating in the clinical trial; and
populate a set of visual elements within a graphical user interface, such that the visual elements indicate the patient burden score, the site burden score, and the cost burden compared to a range of patient burden scores, site burden scores, or the cost burdens from a set of historical clinical studies.

10. The system of claim 9, wherein the set of historical clinical studies share a common operational parameter attribute with the clinical trial.

11. The system of claim 9, wherein the operational parameter is at least one of timing, medications, lab tests, blood tests, examinations, non-invasive procedures, invasive procedure, imaging procedure burden, or self-assessment questionnaire burden.

12. The system of claim 9, wherein the patient burden score corresponds to at least one of demographics, participation logistics, lifestyle factors, caregiver involvement, and procedural burden of a patient associated with the clinical trial.

13. The system of claim 9, wherein receiving the at least one attribute of the clinical trial corresponds to extracting, by the processor, the at least one attribute from an electronic document.

14. The system of claim 9, wherein the visual element corresponds to a procedure performed during the clinical study.

15. The system of claim 9, wherein the visual element includes an indicator for a mean or medium value for patient burden scores, site burden scores, or the cost burdens of the set of historical clinical studies.

16. The system of claim 9, wherein the set of operational parameter attributes associated of the clinical trial corresponds to a set of endpoints for the clinical trial.

17. A system comprising:
a server in communication with an electronic device configured to display a graphical user interface, the server configured to:
receive a set of operational parameter attributes associated of a clinical trial;
execute a computer model to calculate a patient burden score, a site burden score, and a cost burden quantifying a patient's burden and experience participating in the clinical trial; and
populate a set of visual elements within the graphical user interface, such that the visual elements indicate the patient burden score, the site burden score, and the cost burden compared to a range of patient burden scores, site burden scores, or the cost burdens from a set of historical clinical studies.

18. The system of claim 17, wherein the set of historical clinical studies share a common operational parameter attribute with the clinical trial.

19. The system of claim 17, wherein the operational parameter is at least one of timing, medications, lab tests, blood tests, examinations, non-invasive procedures, invasive procedure, imaging procedure burden, or self-assessment questionnaire burden.

20. The system of claim 17, wherein the patient burden score corresponds to at least one of demographics, participation logistics, lifestyle factors, caregiver involvement, and procedural burden of a patient associated with the clinical trial.
